# EUROPÄISCHE PATENTANMELDUNG

(11) **EP 1 586 635 A1**
(43) Veröffentlichungstag der Anmeldung: **19.10.2005**
(21) Anmeldenummer: 05011561.7
(22) Anmeldetag: 22.06.1991
(51) Int. Cl.: C12N 9/64

(54) **Fusionsproteine mit Immunglobulinanteilen, ihre Herstellung und Verwendung**

(30) Priorität: 28.06.1990 DE 4020607
(62) Teilanmeldung aus: 97120664.4
(71) Anmelder: HOECHST AKTIENGESELLSCHAFT, 65929 Frankfurt am Main (DE); THE GENERAL HOSPITAL CORPORATION, Boston, MA 02114 (US)
(72) Erfinder: Lauffer, Leander, Dr., 35075 Gladenbach (DE); Oquendo, Patricia, Dr., 35039 Marburg (DE); Zettlmeissl, Gerd, Dr., 35083 Wetter (DE); Seed, Brian, Dr., Boston, Massachussetts 02114 (US)
(74) Vertreter: Jacobi, Markus Alexander

(57) **Zusammenfassung**

Die Erfindung betrifft gentechnisch erzeugte lösliche Fusionsproteine bestehend aus nicht zur Immunglobulinfamilie gehörigen humanen Proteinen oder Teilen davon und verschiedenen Anteilen der konstanten Region von Immunglobulinmolekülen. Die funktionellen Eigenschaften beider Fusionspartner bleiben überraschenderweise im Fusionsprotein erhalten.

## Beschreibung

Die Erfindung betrifft gentechnisch erzeugte lösliche Fusionsproteine bestehend aus nicht zur Immunglobulinfamilie gehörigen humanen Proteinen oder Teilen davon und verschiedenen Anteilen der konstanten Region von Immunglobulinmolekülen. Die funktionellen Eigenschaften beider Fusionspartner bleiben überraschenderweise im Fusionsprotein erhalten.

Aus der EP-A 0325 262 und der EP-A 0314 317 sind entsprechende Fusionsproteine bestehend aus verschiedenen Domänen des CD4-Membranproteins menschlicher T-Zellen und aus humanen IgGl-Anteilen bekannt. Einige dieser Fusionsproteine binden mit gleicher Affinität an das Glykoprotein gp120 des Humanen Immundefizienz-Virus wie das zellgebundene CD4 Molekül. Das CD4-Molekül gehört zur Immunglobulinfamilie und ist folglich bezüglich seiner Tertiärstruktur sehr ähnlich aufgebaut wie Immunglobulinmoleküle. Dies gilt auch für die α-Kette des T-Zell-Antigenrezeptors, für die solche Fusionen ebenfalls beschrieben wurden (Gascoigne et al., Proc. Natl. Acad. Sci. USA, Bd. 84 (1987), 2937-2940). Aufgrund der sehr ähnlichen Domänenstruktur war deshalb in diesem Fall die Beibehaltung der biologischen Aktivität der beiden Fusionspartner im Fusionsprotein zu erwarten.

Die erfindungsgemäß vorzugsweise an den Aminoterminus der konstanten Region von Immunglobulin gekoppelten humanen Proteine gehören nicht zur Immunglobulinfamilie und sind folgenden Klassen zuzuordnen: (i) membranständige Proteine, deren extrazelluläre Domäne ganz oder teilweise in die Fusion eingebracht wird. Insbesondere sind dies Thromboplastin und Cytokin- und Wachstumsfaktorrezeptoren, wie die zellulären Rezeptoren für Interleukin-4, Interleukin-7, Tumor-Nekrose-Faktor, GM-CSF, G-CSF, Erythropoietin; (ii) nicht membran-ständige lösliche Proteine, die ganz oder teilweise in die Fusion eingebracht werden. Insbesondere sind dies Proteine von therapeutischem Interesse wie z.B. Erythropoietin und andere Cytokine und Wachstumsfaktoren.

Die Fusionsproteine können in bekannten pro- und eukaryontischen Expressionssystemen hergestellt werden, vorzugsweise jedoch in Säugerzellen (z.B. CHO-, COS-, BHK-Zellen).

Die erfindungsgemäßen Fusionsproteine sind aufgrund ihres Immunglobulinanteils mittels Affinitätschromatographie leicht zu reinigen und besitzen verbesserte pharmakokinetische Eigenschaften in vivo.

In vielen Fällen ist der Fc-Teil im Fusionsprotein für den Einsatz in Therapie und Diagnostik durchaus vorteilhaft und führt so z.B. zu verbesserten pharmakokinetischen Eigenschaften (EP-A 0232 262). Andererseits wäre für manche Anwendungen die Möglichkeit einer Entfernung des Fc-Teils wünschenswert, nachdem das Fusionsprotein auf die beschriebene vorteilhafte Art exprimiert, nachgewiesen und gereinigt wurde. Dies ist dann der Fall, wenn sich der Fc-Anteil für den Einsatz in Therapie und Diagnostik als hinderlich erweist, z.B. wenn das Fusionsprotein als Antigen für Immunisierungen dienen soll.

Es existieren verschiedene Proteasen, deren Verwendung für diesen Zweck als denkbar erscheint. Papain oder Pepsin werden beispielsweise für die Erzeugung von F(ab)-Fragmenten aus Immunglobulinen eingesetzt (Immunology, Hrsg. Roitt, I. et al., Gower Medical Publishing, London (1989)), jedoch spalten sie nicht sonderlich spezifisch. Der Blutgerinnungsfaktor Xa hingegen erkennt in einem Protein die relativ seltene Tetrapeptidsequenz Ile-Glu-Gly-Arg und führt eine hydrolytische Spaltung des Proteins nach dem Argininrest durch Spaltsequenzen, die das beschriebene Tetrapeptid enthalten, wurden zuerst von Nagai und Thogersen in ein Hybridprotein auf gentechnologischem Wege eingeführt (Nagai, K. und Thogersen, H.C., Nature, Bd. 309 (1984), 810-812). Diese Autoren konnten zeigen, daß die in E. coli exprimierten Proteine tatsächlich spezifisch von Faktor Xa gespalten werden. Aus Publikationen ist jedoch noch kein Beispiel bekannt, daß solche Proteine auch in eukaryotischen und insbesondere in Animalzellen exprimiert und nach ihrer Reinigung von Faktor Xa gespalten werden können. Eine Expression der erfindungsgemäßen Proteine in Animalzellen ist jedoch vorzuziehen, da nur in einem solchen Zellsystem die Sezernierung von z.B. normalerweise membranständige Rezeptoren als Fusionspartner unter Beibehaltung ihrer nativen Struktur und damit ihrer biologischen Aktivität zu erwarten ist. Sezernierung in den Zellkulturüberstand erleichtert die nachfolgende einfache Reinigung des Fusionsproteins.

Die Erfindung betrifft somit gentechnisch erzeugte lösliche Fusionsproteine bestehend aus nicht zur Immunglobulinfamilie gehörenden humanen Proteine oder Teilen davon und verschiedenen Anteilen der konstanten Regionen von schweren oder leichten Ketten von Immunglobulinen verschiedener Subklassen (IgG, IgM, IgA, IgE). Als Immunglobulin bevorzugt ist der konstante Teil der schweren Kette von humanem IgG, besonders bevorzugt von humanem IgG1, wobei die Fusion an den Hinge-Bereich erfolgt. In einer besonderen Ausführungsform ist der Fc-Teil durch eine miteingebaute mittels Faktor Xa spaltbare Spaltsequenz auf einfache Weise abtrennbar. Ferner betrifft die Erfindung Verfahren zur gentechnischen Herstellung dieser Fusionsproteine sowie deren Verwendung für die Diagnostik und die Therapie.

Die Erfindung ist schließlich in weiteren Beispielen erläutert.

### Beispiel 1: Thromboplastin-Fusionsproteine

Die Blutgerinnung ist ein Vorgang von zentraler Bedeutung im menschlichen Organismus. Entsprechend fein reguliert ist die Gerinnungskaskade, in der eine Vielzahl zellulärer Faktoren und Plasmaproteine zusammenwirken. Die Gesamtheit dieser Proteine (und deren Kofaktoren) wird als Gerinnungsfaktoren bezeichnet. Endprodukte der Gerinnungskaskade sind Thrombin, das die Aggregation von Blutplättchen (Platelets) induziert, und Fibrin, das den Plateletthrombus stabilisiert. Thrombin katalysiert die Bildung von Fibrin aus Fibrinogen und wird selbst durch limitierte Proteolyse von Prothrombin gebildet. Für diesen Schritt ist aktivierter Faktor X (Faktor Xa) verantwortlich, der in Gegenwart von Faktor Va und Calciumionen an Plateletmembranen bindet und Prothrombin spaltet.

Zwei Wege existieren zur Aktivierung von Faktor X, der extrinsische und der intrinsische "pathway". Im intrinsischen "pathway" wird eine Serie von Faktoren durch Proteolyse aktiviert, um jeweils selbst aktive Proteasen zu bilden. Im extrinsischen "pathway" wird Thromboplastin (Tissue Factor) von verletzten Zellen verstärkt synthetisiert und aktiviert Faktor X, zusammen mit Faktor VIIa und Calciumionen. Früher wurde angenommen, daß sich die Aktivität von Thromboplastin auf diese Reaktion beschränkt. Jedoch greift der Thromboplastin/VIIa-Komplex auf der Ebene von Faktor IX ebenfalls aktivierend in den intrinsischen "pathway" ein. Ein Thromboplastin/VIIa-Komplex ist also einer der wichtigsten physiologischen Aktivatoren der Blutgerinnung.

Es ist daher vorstellbar, daß Thromboplastin, abgesehen von seiner Verwendung als Diagnostikum (s.u.), auch als Bestandteil von Therapeutika zur Behandlung angeborener oder erworbener Blutgerinnungsdefizienzen eingesetzt werden kann. Beispiele hierfür sind chronische Hämophilien verursacht durch einen Mangel an Faktoren VIII, IX oder XI oder auch akute Störungen der Blutgerinnung als Folge von z.B. Leber-oder Nierenerkrankungen. Auch nach chirurgischen Eingriffen wäre der Einsatz eines solchen Therapeutikums denkbar.

Thromboplastinist ein integrales Membranprotein, das nicht zur Immunglobulinfamilie gehört. Thromboplastin-cDNA-Sequenzen sind von insgesamt vier Gruppen veröffentlicht worden (Fisher et al., Thromb. Res., Bd. 48 (1987), 89-99; Morrisey et al., Cell, Bd. 50 (1987), 129-135; Scarpati et al., Biochemistry, Bd. 26 (1987), 5234-5238; Spicer et al., Proc. Natl. Acad. Sci. USA, Bd. 84 (1987), 5148-5152). Die Thromboplastin-cDNA beinhaltet einen offenen Leserahmen, der für ein Polypeptid aus 295 Aminosäureresten kodiert, wovon die N-terminalen 32 Aminosäuren als Signalpeptid fungieren. Reifes Thromboplastin besteht aus 263 Aminosäureresten und besitzt eine Drei-Domänen-Struktur: i) aminoterminale extrazelluläre Domäne (219 Aminosäurereste); ii) Transmembranregion (23 Aminosäurereste); iii) cytoplasmatische Domäne (Carboxyterminus; 21 Aminosäurereste). In der extrazellulären Domäne existieren drei potentielle Stellen für N-Glykosylierung (Asn-X-Thr). Thromboplastin ist normalerweise glykosyliert, jedoch scheint die Glykosylierung nicht essentiell für die Aktivität des Proteins zu sein (Paborsky et al., Biochemistry, Bd. 28 (1989), 8072-8077).

Thromboplastin wird als Zusatz zu Plasmaproben in der Gerinnungsdiagnostik benötigt. Durch die einstufige Prothrombin-Gerinnungszeitbestimmung (z.B. Quick-Test) läßt sich der Gerinnungsstatus der untersuchten Person feststellen. Das für die Diagnostik erforderliche Thromboplastin wird gegenwärtig aus humanem Gewebe gewonnen, wobei das Herstellungsverfahren schwer standardisierbar ist, die Ausbeute niedrig liegt und erhebliche Mengen humanes Ausgangsmaterial (Plazenten) bereit gestellt werden müssen. Andererseits ist zu erwarten, daß auch die gentechnische Herstellung von nativem, membrange-bundenem Thromboplastin, bedingt durch komplexe Reinigungsverfahren, problematisch sein wird. Diese Problematiken können durch die erfindungsgemäße Fusion an Immunglobulinanteile umgangen werden. Die erfindungsgemäßen Thromboplastin-Fusionsproteine werden von Säugerzellen (z.B. CHO-, BHK-, COS-Zellen) ins Kulturmedium ausgeschleust, über Affinitätschromatographie an Protein A-Sepharose gereinigt und besitzen überraschend hohe Aktivität in der einstufigen Prothrombin-Gerinnungs-zeitbestimmung.

### Klonierung von Thromboplastin-cDNA

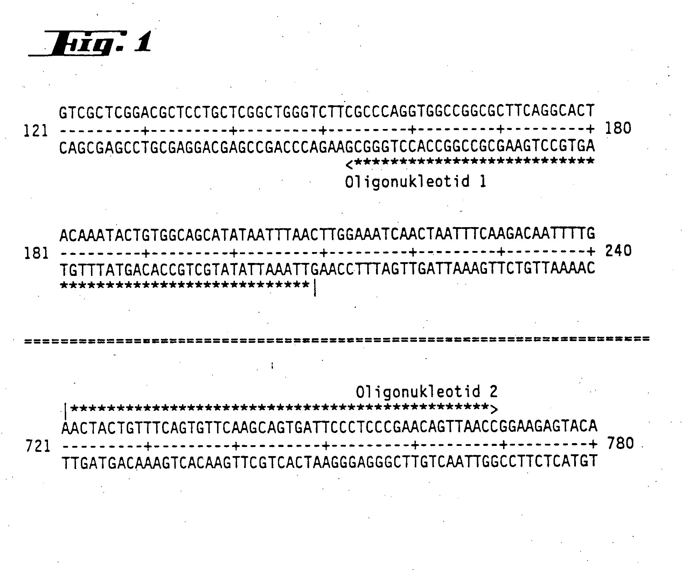

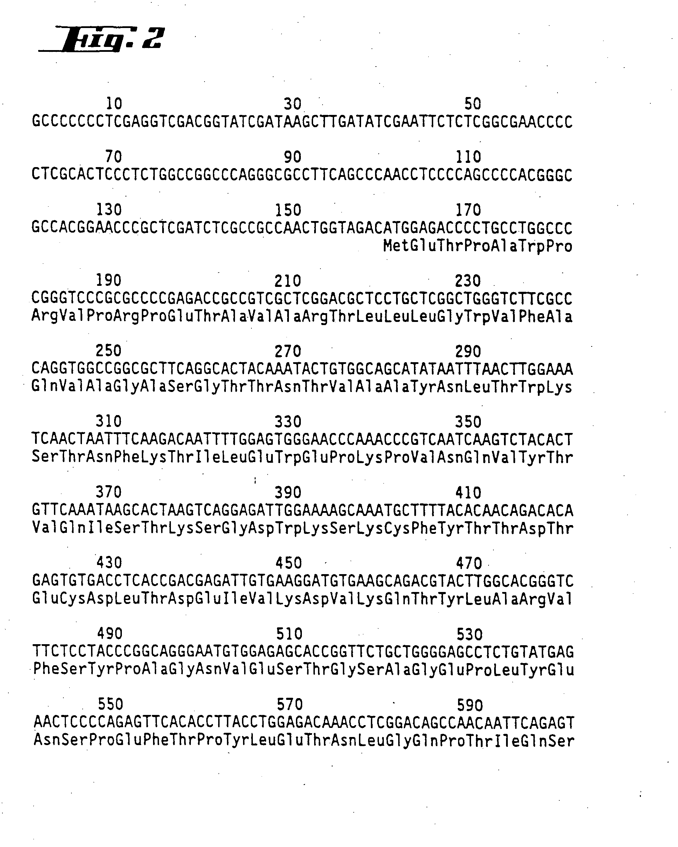

Zur Klonierung der Thromboplastin-cDNA wurde die publizierte Sequenz von Scarpati et al., Biochemistry, Bd. 26 (1987), 5234-5238, benutzt. Hieraus wurden zwei Oligonukleotidsondenmoleküle (s. Fig.1) abgeleitet. Mit diesen beiden Sondenmolekülen wurde eine cDNA-Bank aus humaner Placenta (Grundmann et al., Proc. Natl. Acad. Sci. USA, Bd. 83 (1986), 8024-8028) abgesucht.

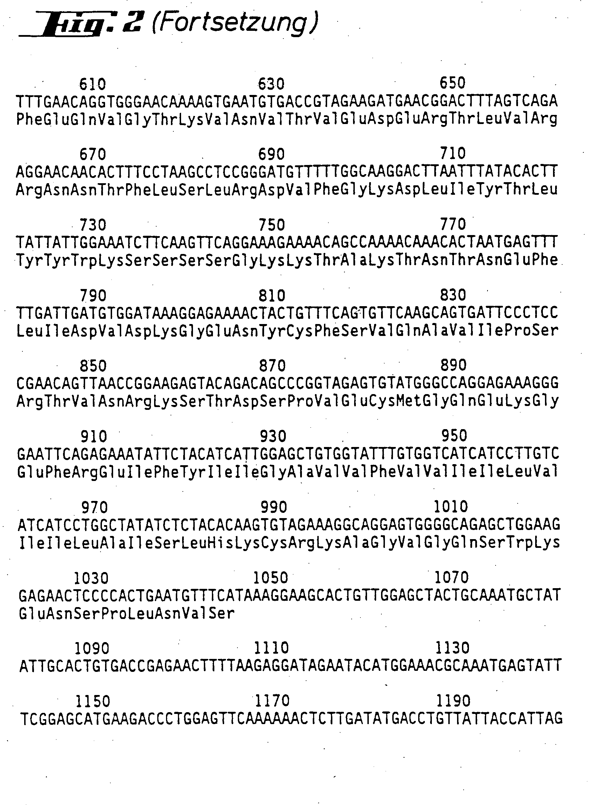

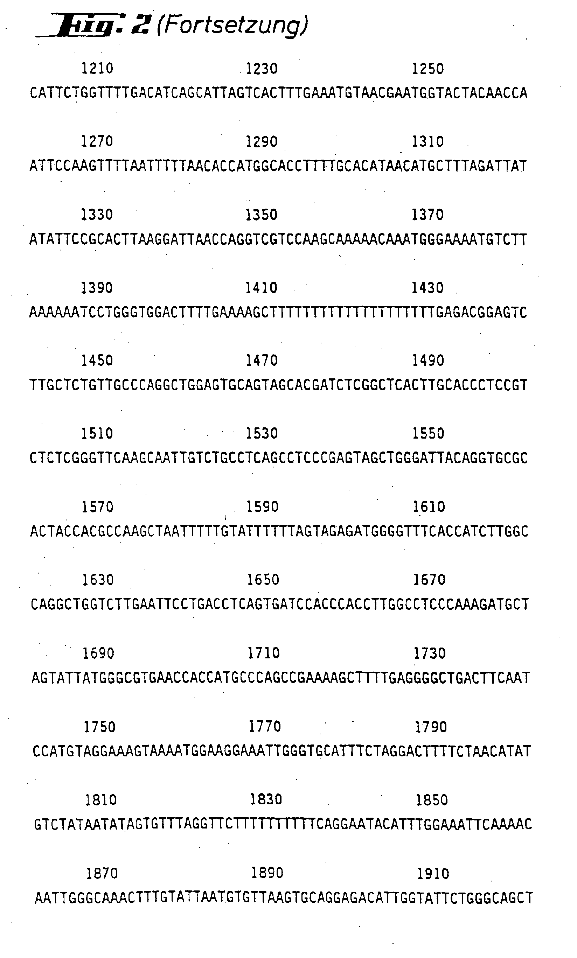

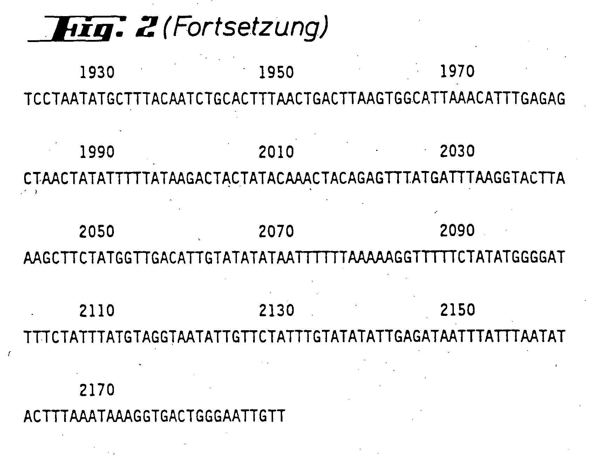

Es wurden verschieden lange cDNA-Klone erhalten. Ein Klon, 2b-Apr5, der für das weitere Vorgehen verwendet wird, kodiert für die gleiche Aminosäuresequenz, wie die in Scarpati et al. beschriebene cDNA. In Fig. 2 ist die Gesamtsequenz des Klons 2b-Apr5 mit der daraus abgeleiteten Thromboplastin-Aminosäuresequenz dargestellt.

### Konstruktion eines für Thromboplastin-Fusionsprotein kodierenden Hybridplasmids pTF1Fc.

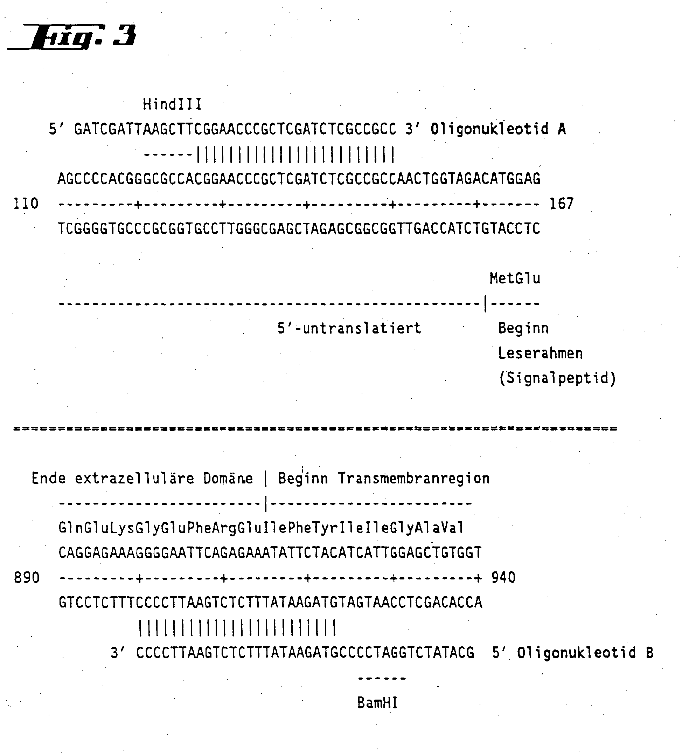

Das Plasmid pCD4E gamma 1 (EP 0 325 262 A2; hinterlegt bei ATCC unter der Nummer No. 67610) dient zur Expression eines Fusionsproteins aus humanem CD4-Rezeptor und humanem IgGl. Die für die extrazelluläre Domäne von CD4 kodierende DNA-Sequenz wird aus diesem Plasmid mit den Restriktionsenzymen HindIII und BamHI entfernt. Mit dem Enzym HindIII darf hierbei nur eine teilweise Spaltung durchgeführt werden, um nur bei einer der zwei in pCD4E gamma 1 enthaltenen HindIII-Stellen zu schneiden (Position 2198). Es liegt dann ein geöffneter Vektor vor, bei dem eine eukaryotische Transkriptionsregulationssequenz (Promotor) von der offenen HindIII-Stelle gefolgt wird. Die offene BamHI-Stelle liegt am Beginn der kodierenden Regionen für einen Pentapeptidlinker, gefolgt von den Hinge- und den CH2- und CH3-Domänen vom menschlichem IgGl. Der Leserahmen in der BamHI-Erkennungssequenz GGATCC ist dergestalt, daß GAT als Asparaginsäure translatiert wird. DNA-Amplifizierung mit thermostabiler DNA-Polymerase ermöglicht eine gegebene Sequenz so zu verändern, daß an eines oder beide Enden beliebige Sequenzen angefügt werden. Zwei Oligonukleotide wurden synthetisiert, die mit Sequenzen in der 5'-untranslatierten Region
(A: 5' GATCGATTAAGCTTCGGAACCCGCTCGATCTCGCCGCC 3') bzw. kodierenden Region
(B: 5' GCATATCTGGATCCCCGTAGAATATTTCTCTGAATTCCCC 3') der Thromboplastin-cDNA hybridisieren können. Oligonukleotid A ist dabei teilweise homolog zur Sequenz des kodierenden stranges, Oligonukleotid B ist teilweise homolog zum nicht-kodierenden Strang; vergl. Fig. 3.

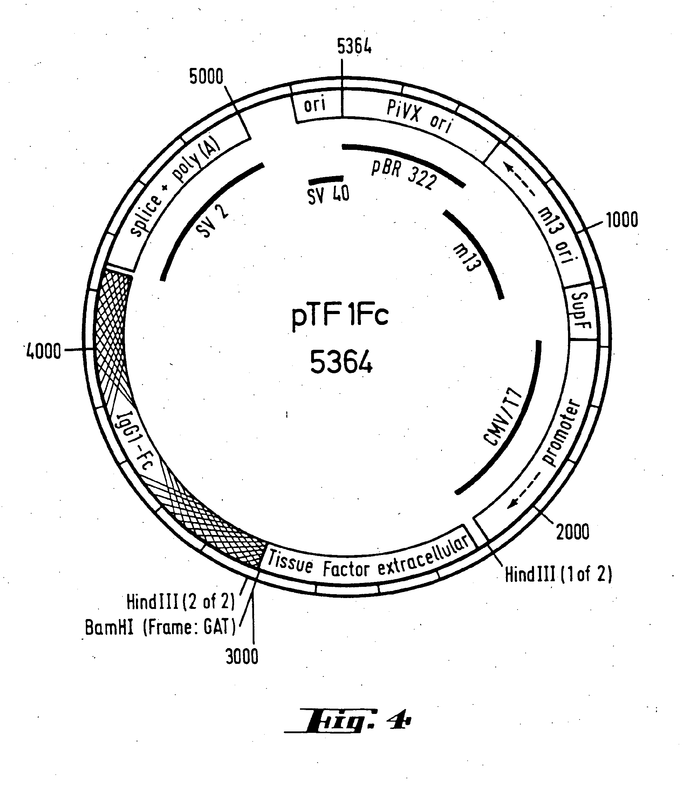

Nach erfolgter Amplifizierung liegt also ein DNA-Fragment vor (827 bp), das (bezogen auf den kodierenden Strang) am 5'-Ende vor dem Beginn der kodierenden Sequenz eine HindIII-Stelle, am 3'-Ende nach den Kodons für die ersten drei Aminosäurereste der Transmembranregion eine BamHI-Stelle enthält. Der Leserahmen in der BamHI-Schnittstelle ist dergestalt, daß nach Ligierung mit der BamHI-Stelle in pCD4E gamma 1 eine Genfusion erreicht wird mit einem durchgehenden Leserahmen vom Initiationskodon der Thromboplastin-cDNA bis zum Stopkodon der schweren Kette des IgG1. Das erwünschte Fragment wurde erhalten und nach Behandlung mit HindIII und BamHI in den oben beschriebenen mit HindIII (partiell)/BamHI geschnittenen Vektor pCD4E gamma 1 ligiert. Das resultierende Plasmid erhielt den Namen pTF1Fc (Fig. 4).

### Transfektion von pTF1Fc in Säugerzellen

Das durch das Plasmid pTF1Fc kodierte Fusionsprotein wird im folgenden als pTF1Fc bezeichnet. pTF1Fc wurde transient in COS-Zellen exprimiert. Hierzu wurden COS-Zellen mit Hilfe von DEAE-Dextran mit pTF1Fc transfiziert (EP A 0325 262).

Indirekte Immunfluoreszenzuntersuchungen ergaben ca. 25% als Anteil transfizierter Zellen. 24 h nach Transfektion wurden die Zellen in serumfreies Medium überführt. Dieser Zellüberstand wurde nach weiteren drei Tagen geerntet.

### Reinigung von pTF1Fc-Fusionsprotein aus Zellkulturüberständen

170 ml Überstand von transient transfizierten COS-Zellen wurden über Nacht in einem Batchprozeß bei 4°C mit 0,8 ml Protein-A-Sepharose in einer Säule gesammelt, mit 10 Volumen Waschpuffer gewaschen (50mM Tris-Puffer pH 8,6, 150mM NaCl) und mit Elutionspuffer (100mM Citronensäure: 100mM NaCitrat 93:7) in 0,5 ml Fraktionen eluiert. Die ersten 9 Fraktionen wurden nach sofortiger Neutralisierung mit jeweils 0,1 ml 2M Tris-Puffer pH 8,6 vereinigt und das enthaltene Protein durch drei Zyklen Konzentration/Verdünnung im Amicon-Mikrokonzentrator (Centricon 30) in TNE-Puffer (50mM Tris-Puffer pH 7,4, 50mM NaCl, 1mM EDTA) überführt. Das so erhaltene pTF1Fc ist in der SDS-PAGE elektrophoretisch rein (U.K. Lämmli, Nature 227 (1970) 680-685). In Abwesenheit von Reduktionsmitteln verhält es sich in der SDS-PAGE wie ein Dimer (ca. 165 KDa).

### Biologische Aktivität von gereinigtem TF1Fc in der Prothrombin-Gerinnungs-zeitbestimmung

TF1Fc-Fusionsprotein ist in niedrigen Konzentrationen (> 50 ng/ml) in der einstufigen Prothrombin-Gerinnungszeitbestimmung (Vinazzer, H. Gerinnungsphysiologie und Methoden im Blutgerinnungslabor (1979), Fisher Verlag, Stuttgart) aktiv. Die erzielten Gerinnungszeiten sind vergleichbar mit den Gerinnungszeiten, die mit Thromboplastin, das aus humaner Plazenta isoliert wurde, erhalten werden.

### Beispiel 2: Interleukin-4-Rezeptor-Fusionsproteine

Interleukin-4 (IL-4) wird von T-Zellen synthetisiert und wurde ursprünglich als B-Zell-Wachstumsfaktor bezeichnet, da es B-Zell-Proliferation stimulieren kann. Es übt eine Vielzahl von Effekten auf diese Zellen aus. Insbesondere ist dies das Anregen der Synthese von Molekülen der Immunglobulinsubklassen IgGl und IgE in aktivierten B-Zellen (Coffmann et al., Immunol. Rev., Bd. 102 (1988) 5). Darüber hinaus reguliert IL-4 auch die Proliferation und Differenzierung von T-Zellen und anderen haematopoetischen Zellen. Es trägt somit zur Regulierung von allergischen und anderen immunologischen Reaktionen bei. IL-4 bindet mit hoher Affinität an einen spezifischen Rezeptor. Die cDNA, die für den humanen IL-4-Rezeptor kodiert, wurde isoliert (Idzerda et al., J.Exp.Med. Bd. 171 (1990) 861-873. Aus der Analyse der von der cDNA Sequenz abgeleiteten Aminosäuresequenz geht hervor, daß der IL-4 Rezeptor aus insgesamt 825 Aminosäuren besteht, wobei die N-terminalen 25 Aminosäuren als Signalpeptid fungieren. Reifer humaner IL-4-Rezeptor besteht aus 800 Aminosäuren und besitzt wie Thromboplastin eine Dreidomänenstruktur: i) aminoterminale extrazelluläre Domäne (207 Aminosäuren); ii) Transmembranregion (24 Aminosäuren) und iii) cytoplasmatische Domäne (569 Aminosäuren). In der extrazellulären Domäne existieren sechs potentielle Stellen für N-Glykosylierung (Asn-X-Thr/Ser). IL-4-Rezeptor besitzt Homologien zum humanen I1-6-Rezeptor, zur ß-Untereinheit des humanen IL-2-Rezeptors, zum Maus-Erythropoietin-Rezeptor und zum Ratten-Prolaktinrezeptor (Idzerda et al., a.a.o.). Es ist somit wie Thromboplastin kein Mitglied der Immunglobulinfamilie, sondern wird zusammen mit den aufgeführten homologen Proteinen zur neuen Familie der Haematopoetinrezeptoren gerechnet. Mitgliedern dieser Familie sind 4 cysteinreste und eine sich in der Nähe der Transmembranregion befindliche konservierte Sequenz (Trp-Ser-X-Trp-Ser) in der extrazellulären Domäne gemeinsam.

Aufgrund der beschriebenen Funktion des IL-4/IL-4-Rezeptor-systems ist ein therapeutischer Einsatz einer rekombinanten Form des IL-4-Rezeptors zur Unterdrückung IL-4-vermittelter Immunreaktionen (z.B. Transplantationsabstoßungsreaktion, Autoimmunkrankheiten, allergische Reaktionen) möglich.

Die für eine Therapie erforderliche Substanzmenge machen eine gentechnische Herstellung solcher Moleküle notwendig. Erfindungsgemäß ist wegen der problemlosen Reinigung durch Affinitätschromatographie und der verbesserten pharmakokinetischen Eigenschaften die Synthese von löslichen Formen des IL-4-Rezeptors als Immunglobulinfusionsprotein besonders vorteilhaft.

Die IL-4-Rezeptor-Fusionsproteine werden von Säugerzellen (z.B. CHO-, BHK-, COS-Zellen) in das Kulturmedium ausgeschleust, über Affinitätschromatographie an Protein-A-Sepharose gereinigt und besitzen überraschenderweise identische funktionelle Eigenschaften wie die extrazelluläre Domäne des intakten membrangebundenen IL-4-Rezeptormoleküls.

### Konstruktion eines für IL-4-Rezeptor-Fusionsprotein kodierenden Hybridplasmids pIL-4RFc.

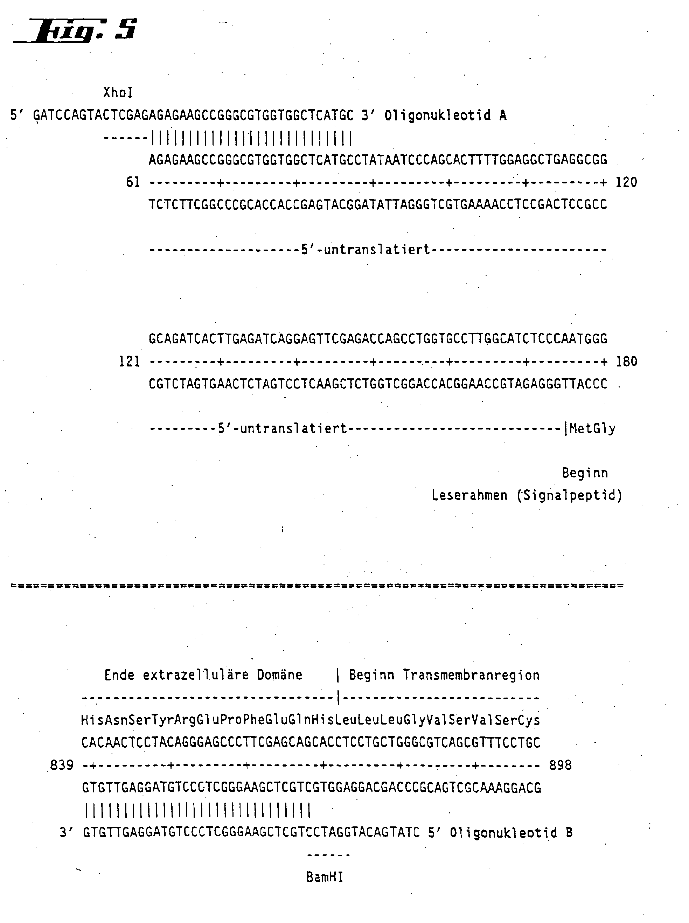

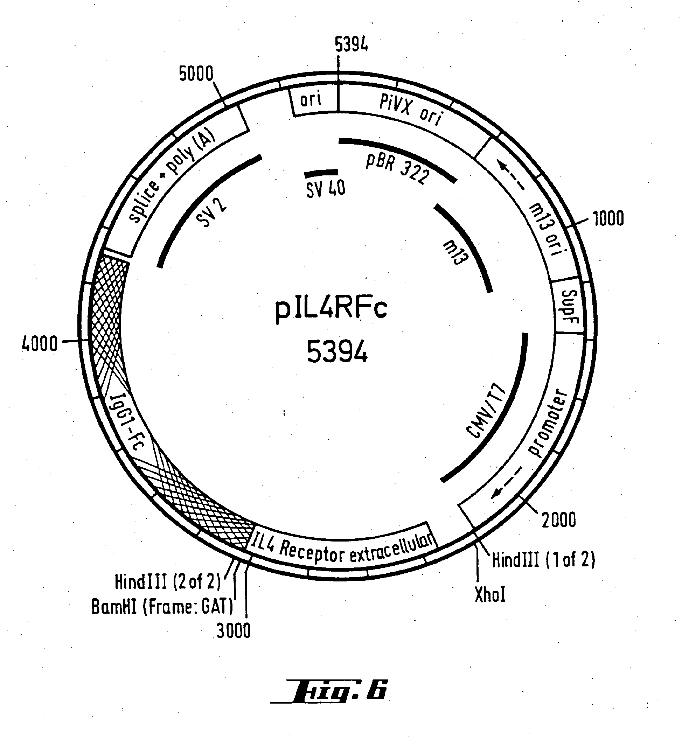

Wird das Plasmid pCD4EGammal mit XhoI und BamHI geschnitten, liegt ein geöffneter Vektor vor, bei dem die offene XhoI-Stelle "downstream" von der Promotersequenz liegt. Die offene BamHI-Stelle liegt am Beginn der kodierenden Regionen für einen Pentapeptidlinker, gefolgt von den Hinge- und den CH2- und CH3-Domänen vom menschlichem IgGl. Der Leserahmen in der BamHI-Erkennungssequenz GGATCC ist dergestalt, daß GAT als Asparaginsäure translatiert wird. DNA-Amplifizierung mit thermostabiler DNA-Polymerase ermöglicht eine gegebene Sequenz so zu verändern, daß an eines oder beide Enden beliebige Sequenzen angefügt werden. Zwei Oligonukleotide wurden synthetisiert, die mit Sequenzen in der 5'-untranslatierten Region
(A: 5' GATCCAGTACTCGAGAGAGAAGCCGGGCGTGGTGGCTCATGC 3') bzw. kodierenden Region
(B: 5' CTATGACATGGATCCTGCTCGAAGGGCTCCCTGTAGGAGTTGTG 3') der IL-4-Rezeptor-cDNA, die kloniert im Vektor pDC302/T22-8 vorliegt (Idzerda et al., a.a.O.), hybridisieren können. Oligonukleotid A ist dabei teilweise homolog zur Sequenz des kodierenden Stranges, Oligonukleotid B ist teilweise homolog zum nicht-kodierenden Strang; vergl. Fig. 5. Nach erfolgter Amplifizierung mittels thermostabiler DNA-Polymerase liegt ein DNA-Fragment vor (836 bp), das bezogen auf den kodierenden Strang am 5'-Ende vor dem Beginn der kodierenden Sequenz eine XhoI-Stelle, am 3'-Ende vor dem letzten Kodon der extrazellulären Domäne eine BamHI-Stelle enthält. Der Leserahmen in der BamHI-Schnittstelle ist dergestalt, daß nach Ligierung mit der BamHI-Stelle in pCD4E gamma-1 eine Genfusion erreicht wird mit einem durchgehenden Leserahmen vom Initiationskodon der IL-4-Rezeptor-cDNA bis zum Stopkodon der schweren Kette des IgG1. Das erwünschte Fragment wurde erhalten und nach Behandlung mit XhoI und BamHI in den oben beschriebenen mit XhoI/BamHI geschnittenen Vektor pCD4E gamma 1 ligiert. Das resultierende Plasmid erhielt den Namen pIL4RFc (Fig. 6).

### Transfektion von pIL4RFc in Säugerzellen

Das durch das Plasmid pIL4RFc kodierte Fusionsprotein wird im folgenden als pIL4RFc bezeichnet. pIL4RFc wurde transient in COS-Zellen exprimiert. Hierzu wurden COS-Zellen mit Hilfe von DEAE-Dextran mit pIL4RFc transfiziert (EP A 0325 262). Indirekte Immunfluoreszenzuntersuchungen ergaben ca. 25% als Anteil transfizierter Zellen. 24 h nach Transfektion wurden die Zellen in serumfreies Medium überführt. Dieser Zellüberstand wurde nach weiteren drei Tagen geerntet.

### Reinigung von IL4RFc-Fusionsprotein aus Zellkulturüberständen

500 ml Überstand von transient transfizierten COS-Zellen wurden über Nacht in einem Batchprozeß bei 4°C mit 1,6 ml Protein-A-Sepharose in einer Säule gesammelt, mit 10 Volumen Waschpuffer gewaschen (50mM Tris-Puffer pH 8,6, 150mM NaCl) und mit Elutionspuffer (100mM Citronensäure: 100mM NaCitrat 93:7) in 0,5 ml Fraktionen eluiert. Die ersten 9 Fraktionen wurden nach sofortiger Neutralisierung mit jeweils 0,1 ml 2M Tris-Puffer pH 8,6 vereinigt und das enthaltene Protein durch drei Zyklen Konzentration/Verdünnung im Amicon-Mikrokonzentrator (Centricon 30) in TNE-Puffer (50mM Tris-Puffer pH 7,4, 50mM NaCl, 1mM EDTA) überführt. Das so erhaltene IL4RFc ist in der SDS-PAGE elektrophoretisch rein (U.K. Lämmli, Nature 227 (1970) 680-685). In Abwesenheit von Reduktionsmitteln verhält es sich in der SDS-PAGE wie ein Dimer (ca. 150 KDa).

### Biologische Aktivität von gereinigtem IL4RFc

IL4RFc-Protein bindet ¹²⁵I-radiomarkiertes IL-4 mit gleicher Affinität (KD=0.5nM) wie membrangebundener, intakter IL-4-Rezeptor. Es hemmt die Proliferation der IL-4-abhängigen Zellinie CTLLHuIL-4RI Klon D (Idzerda et al., a.a.O.) in Konzentrationen von 10-1000 ng/ml. Darüber hinaus eignet es sich hervorragend für die Entwicklung von IL-4-Bindungstests, da es über seinen Fc-Teil an mit z.B. Kaninchen-anti-human-IgG vorbeschichtete Mikrotiterplatten gebunden werden kann und in dieser Form ebenfalls mit hoher Affinität seinen Liganden bindet.

### Beispiel 3: Erythropoietin-Fusionsproteine

Reifes Erythropoietin (EPO) ist ein aus 166 Aminosäuren bestehendes Glykoprotein, das essentiell für die Entwicklung von Erythrozyten ist. Es stimuliert die Reifung und die terminale Differenzierung erythroider Vorläuferzellen. Die cDNA für humanes EPO wurde kloniert (EPA-0267 678) und kodiert für die 166 Aminosäuren des reifen EPO und ein für die Sezernierung essentielles Signalpeptid von 22 Aminosäuren. Mit Hilfe der cDNA kann rekombinantes funktionelles EPO in gentechnisch veränderten Säugerzellen hergestellt werden und zur Therapie von anämischen Erscheinungen verschiedenen Ursprungs (z.B. bei akuten Nierenversagen) klinisch eingesetzt werden.

Erfindungsgemäß ist wegen der problemlosen Reinigung und der verbesserten pharmakokinetischen Eigenschaften die Synthese von EPO als Immunglobulinfusionsprotein besonders vorteilhaft.

### Konstruktion eines für Erythropoietin-Fusionsprotein kodierenden Hybridplasmids pEPOFc.

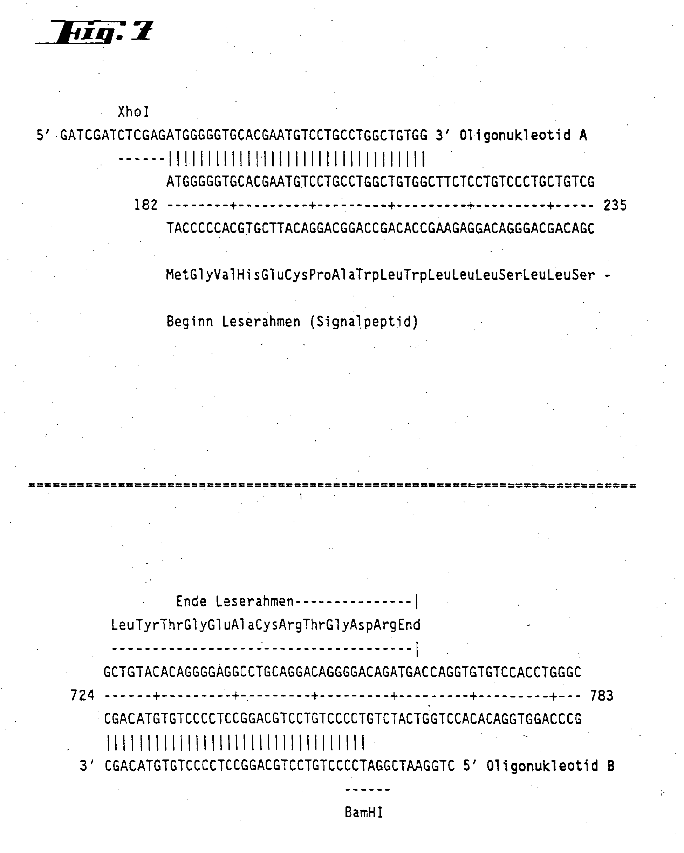

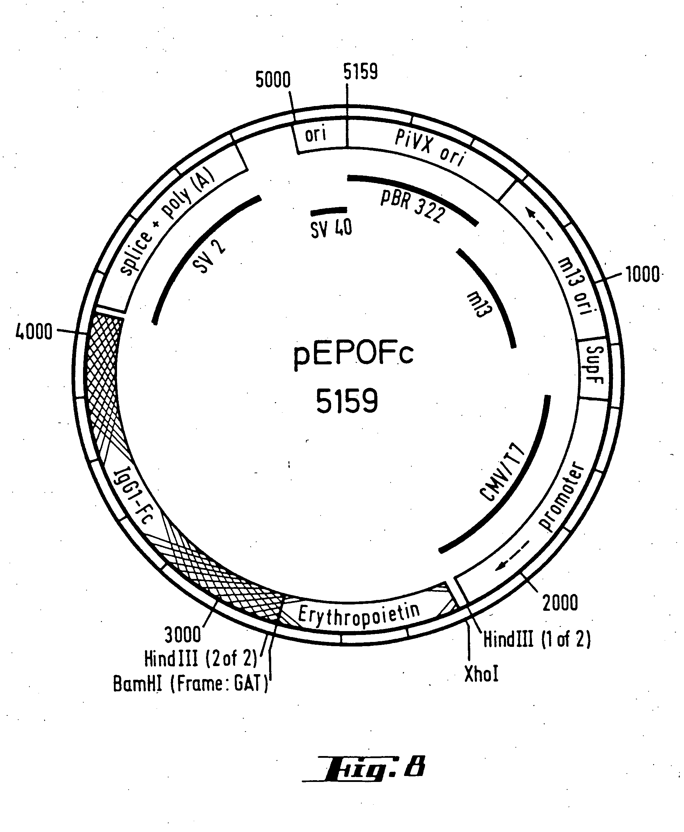

Diese Konstruktion erfolgte analog zu der im Beispiel 2 beschriebenen (Abschnitt: "Konstruktion eines für IL-4-Rezeptor-Fusionsprotein kodierenden Hybridplasmids pIL-4RFc"). Zwei Oligonukleotide wurden synthetisiert, die mit Sequenzen in der Nähe des Initiationskodons
(A: 5'GATCGATCTCGAGATGGGGGTGCACGAATGTCCTGCCTGGCTGTGG 3') bzw. des Stopkodons
(B: 5' CTGGAATCGGATCCCCTGTCCTGCAGGCCTCCCCTGTGTACAGC 3') der im Vektor pCES klonierten EPO-cDNA (EP A 0267 678) hybridisieren können. Oligonukleotid A ist dabei teilweise homolog zur Sequenz des kodierenden Stranges, Oligonukleotid B ist teilweise homolog zum nicht-kodierenden strang; vergl. Fig. 7. Nach erfolgter Amplifizierung liegt mittels thermostabiler DNA-Polymerase ein DNA-Fragment vor (598 bp), das bezogen auf den kodierenden Strang am 5'-Ende vor dem Initiationskodon eine XhoI-Stelle enthält und in dem am 3'-Ende das Kodon für den vorletzten C-terminalen Aminosäurerest von EPO (Asp) in einer BamHI-Erkennungssequenz vorliegt. Der Leserahmen in der BamHI-Schnittstelle ist dergestalt, daß nach Ligierung mit der BamHI-Stelle in pCD4E gamma 1 eine Genfusion erreicht wird mit einem durchgehenden Leserahmen vom Initiationskodon der EPO-cDNA bis zum Stopkodon der schweren Kette des IgG1. Das erwünschte Fragment wurde erhalten und nach Behandlung mit XhoI und BamHI in den oben beschriebenen mit XhoI/BamHI geschnittenen Vektor pCD4E gamma 1 ligiert. Das resultierende Plasmid erhielt den Namen pEPOFc (Fig. 8).

## Patentansprüche

1. Lösliche Fusionsproteine bestehend aus nicht zur Immunglobulinfamilie gehörigen humanen Proteinen oder Teilen davon und verschiedenen Anteilen von Immunglobulinmolekülen aller Subklassen.

2. Fusionsproteine nach Anspruch 1, **dadurch gekennzeichnet, daß** der Immunglobulinanteil der konstante Teil der schweren Kette von humanem IgG ist.

3. Fusionsproteine nach Anspruch 2, **dadurch gekennzeichnet, daß** der Immunglobulinanteil der konstante Teil der schweren Kette von humanem IgG1 oder ein Protein-A-bindendes Fragment davon ist.

4. Fusionsproteine nach Anspruch 2 oder Anspruch 3, **dadurch gekennzeichnet, daß** die Fusion an die "Hinge"-Region erfolgt.

5. Fusionsproteine nach Ansprüchen 1-4, **dadurch gekennzeichnet, daß** das an Immunglobulin fusionierte Protein der extrazelluläre Anteil eines Membranproteins oder Teilen davon ist.

6. Fusionsproteine nach Ansprüchen 1-4, **dadurch gekennzeichnet, daß** das an Immunglobulin fusionierte Protein der extrazelluläre Anteil von Thromboplastin oder Teilen davon ist.

7. Fusionsproteine nach Ansprüchen 1-4, **dadurch gekennzeichnet, daß** das an Immunglobulin fusionierte Protein der extrazelluläre Anteil eines Cytokin- oder Wachstumsfaktor-Rezeptors oder Teilen davon ist.

8. Fusionsprotein nach Anspruch 7, **dadurch gekennzeichnet, daß** das an Immunglobulin fusionierte Protein der extrazelluläre Anteil von IL-7-Rezeptor oder Teilen davon ist.

9. Fusionsprotein nach Anspruch 7, **dadurch gekennzeichnet, daß** das an Immunglobulin fusionierte Protein der extrazelluläre Anteil von G-CSF-Rezeptor oder Teilen davon ist.

10. Fusionsprotein nach Anspruch 7, **dadurch gekennzeichnet, daß** das an Immunglobulin fusionierte Protein der extrazelluläre Anteil von GM-CSF-Rezeptor oder Teilen davon ist.

11. Fusionsprotein nach Anspruch 7, **dadurch gekennzeichnet, daß** das an Immunglobulin fusionierte Protein der extrazelluläre Anteil von Erythropoietin-Rezeptor oder Teilen davon ist.

12. Fusionsprotein nach Ansprüchen 1-4, **dadurch gekennzeichnet, daß** das an Immunglobulin fusionierte Protein ein nicht membranständiges lösliches Protein oder Teil davon ist.

13. Fusionsprotein nach Anspruch 12, **dadurch gekennzeichnet, daß** das an Immunglobulin fusionierte Protein ein Cytokin- oder Wachstumsfaktor oder Teil davon ist.

14. Fusionsprotein nach Anspruch 13, **dadurch gekennzeichnet, daß** das an Immunglobulin fusionierte Protein Erythropoietin oder Teil davon ist.

15. Fusionsprotein nach Anspruch 13, **dadurch gekennzeichnet, daß** das an Immunglobulin fusionierte Protein GM-CSF oder G-CSF bzw. Teil davon ist.

16. Fusionsprotein nach Anspruch 13, **dadurch gekennzeichnet, daß** das an Immunglobulin fusionierte Protein Interleukin IL-1 bis IL-8 bzw. Teil davon ist.

17. Fusionsprotein nach einem der vorausgehenden Ansprüche 1- 16, **dadurch gekennzeichnet, daß** zusätzlich eine Faktor Xa Spaltstelle zwischen dem Immunglobulinteil und dem Nicht-Immunglobulinteil insertiert ist.

18. Verfahren zur Herstellung von Fusionsproteinen nach einem der Ansprüche 1-17, **dadurch gekennzeichnet, daß** die für diese Konstrukte codierende DNA in ein Säugerzell-Expressionssystem eingebracht und nach Expression das gebildete Fusionsprotein mittels Affinitätschromatographie über den Immunglobulinanteil gereinigt wird.

19. Verwendung der Fusionsproteine nach einem der Ansprüche 1-17 zur Diagnostik.

20. Verwendung der Fusionsproteine nach einem der Ansprüche 1-17 zur Therapie.
